# EUROPEAN PATENT SPECIFICATION

(11) **EP 3 256 456 B1**
(45) Date of publication and mention of the grant of the patent: **11.12.2019**
(21) Application number: 16703779.5
(22) Date of filing: 10.02.2016
(51) Int. Cl.: C07D 309/04

(54) **PROCESS OF PRODUCTION OF CYCLO-DEHYDROLINALOOL (II)**
VERFAHREN ZUR HERSTELLUNG VON CYCLO-DEHYDROLINALOOL (II)
PROCÉDÉ DE PRODUCTION DE CYCLO-DÉSHYDROLINALOOL (II)

(30) Priority: 10.02.2015 EP 15154529
(43) Date of publication of application: 20.12.2017
(73) Proprietor: DSM IP Assets B.V., 6411 TE Heerlen (NL)
(72) Inventor: BEUMER, Raphael, 4303 Kaiseraugst (CH); BONRATH, Werner, 4303 Kaiseraugst (CH); TSCHUMI, Johannes, 4303 Kaiseraugst (CH)
(74) Representative: Steck, Melanie
(86) International application number: PCT/EP2016/052763
(87) International publication number: WO 2016/128423

(56) References cited:
- EP-A1- 1 186 600
- BE-A1- 852 918
- SONI A. SINGH ET AL: "Amberlyst-15-Catalyzed Efficient Cyclization of [gamma]- and [delta]-Unsaturated Alcohols: Green Synthesis of Oxygen Heterocycles", SYNTHETIC COMMUNICATIONS, vol. 40, no. 1, 15 December 2009 (2009-12-15), pages 74-80, XP055181826, ISSN: 0039-7911, DOI: 10.1080/00397910902945345

## Description

The present invention relates to an improved process of producing cyclodehydrolinalool.

Cyclo-dehydrolinalool (c-DLL) can be used for example as an intermediate in the production of 2-vinyl-2,6,6-trimethyl-3,4,5,6-tetrahydro-2H-pyran, which is also known as limetol® (compound of formula (III)) 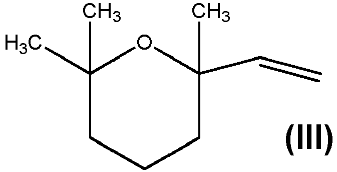 and which is an important and widely used fragrance ingredient.

Due to the importance of 2-vinyl-2,6,6-trimethyl-3,4,5,6-tetrahydro-2H-pyran, there is always a need for improved methods for its production as well as the production of its intermediates.
A way to obtain 2-vinyl-2,6,6-trimethyl-3,4,5,6-tetrahydro-2H-pyran is the reduction of cyclo-dehydrolinalool (compound of formula (I)) 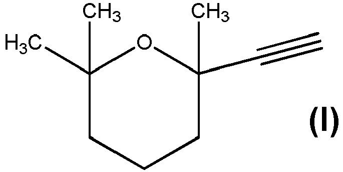 by hydrogenation (for example by using a Lindlar-type catalyst).

This hydrogenation can be carried out with excellent yield and therefore, it was a goal of the present invention to find an improved method to produce cyclo-dehydrolinalool (c-DLL).

It is known form the prior art to produce c-DLL from dehydrolinalool (compound of formula (II)) 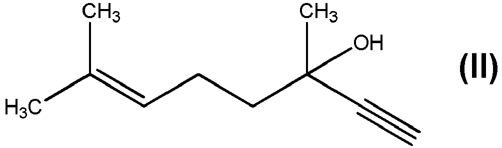 by cyclisation:

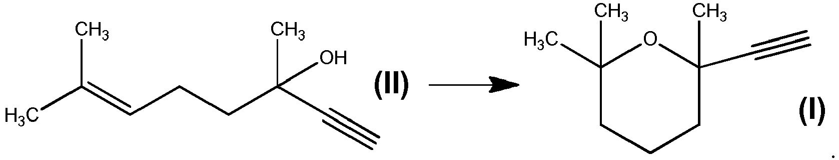

In BE852918 it is disclosed that the cyclisation of dehydrolinalool (DLL) is carried out in the presence of polyphosphoric acid. In the reaction mixture, the molar ratio of DLL to polyphosphoric acid is 10:1. The yield of c-DLL is up to 67.2 %.

As stated above, due to the importance of c-DLL, there is a need for improved production of c-DLL.

Surprisingly, it was found that when the cyclization of DLL is carried in the presence of
(i) at least one polymeric acid containing one or more sulfur (VI) atom as a catalyst and
(ii) at least one inert solvent.

By the term *"polymeric acid"* it is meant that the acid, which is used as catalyst comprises a large molecule or macromolecule, which are composed of many repeated subunits. More specifically the catalysts are acidic ion exchangers. Therefore the present invention relates to a process (P) for the production of the compound of formula (I) 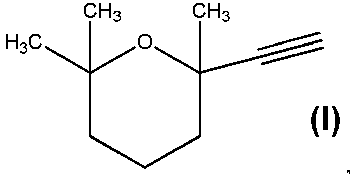 by the cyclization of the compound of formula (II) 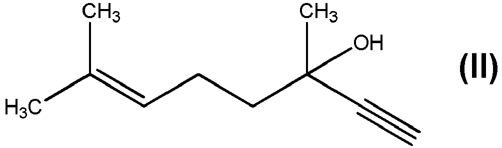 characterized in that the reaction is carried out in at least one inert solvent and in the presence of
(i) at least one acidic ion exchanger containing one or more sulfur (VI) atom as a catalyst and
(ii) at least one inert solvent, wherein a weight ratio of 20:1 to 100:1 of compound (II) to acidic ion exchanger is used.

The product of process (P), which is the compound of formula (I), is obtained in excellent yields. Usually more than 90% yield is obtained. This is a surprising and significant improvement in view of the prior art.

Furthermore the process according to the present invention has also a good heat exchange (allows safe reaction conditions in industry, when produced in industrial scale) and there is no need to add large amount of the catalyst.

Therefore the new process has many advantages, which are important, over the known process to produce the compound of formula (I).

The process according to the present invention is always carried in the presence of at least one acidic ion exchanger containing one or more sulfur (VI) atom as a catalyst and with at least one inert solvent.

The sulfur atom (or the sulfur atoms) is usually and preferably part of -SO₃⁻ or SO₄⁻.

The acid, which is used as a catalyst, is an acidic ion exchanger. Such acidic ion exchangers are commercially available. Suitable acidic ion exchangers for the process according to the present invention are for example:
- acidic Amberlyst (such as Amberlyst®15, Amberlyst®15 dry, Amberlyst®16, Amberlyst®35, Amberlyst®35 dry, Amberlyst®36, Amberlyst®36 dry and Amberlyst®70 from Dow);
- acidic Lewatit® (from Lanxess)
- acidic Dowex® (such as Dowex® 88 MB dry, Dowex® DR2030 from Dow)

As stated above the amount of the acidic ion exchangers which is necessary to obtain the product in excellent yields are low (in comparison to the processes known from the prior art).
Usually a ratio of 20:1 of compound (II) to acidic ion exchanger is used. The ratio is weight related.
Preferably the ratio can be as low as 100:1. This means that a suitable ratio of compound (II) to acid is from 20:1 to 100:1.

Therefore the present invention also relates to a process (P1), which is the process (P), wherein a ratio of 20:1 to 100:1 of compound (II) to acidic ion exchanger is used.

The process according to the present invention is carried in an inert solvent or in a mixture of inert solvents.

The inert solvents are usually aliphatic hydrocarbons or aromatic hydrocarbons. These aliphatic hydrocarbons or aromatic hydrocarbons need to be liquid at the reaction conditions. The hydrocarbons can be linear, branched and/or cyclic.

Therefore the present invention relates to a process (P2), which is the process (P) or (P1), wherein the solvent (or solvents) is chosen from the group consisting of aliphatic hydrocarbons and aromatic hydrocarbons.

Suitable solvents are for example n-hexane, cyclohexane, n-heptane, pentane and toluene.

Therefore the present invention relates to a process (P3), which is the process (P), (P1) or (P2), wherein the solvent (or more than one) is chosen from the group consisting of n-hexane, cyclohexane, n-heptane, pentane and toluene.

The amount of the solvent (or solvent mixture) is not critical for the invention. It is used in usual amounts. The solvents (or solvent mixture) can be added in an equimolar amounts in regard to DLL (compound of formula (II)). But it is also possible to use more as well to use less solvent (or solvent mixture). A preferred range of the ratio of solvent (or solvent mixture) to DLL is 3:1 to 0.5 :1. The ratio is weight related.
Therefore the present invention relates to a process (P4), which is the process (P), (P1), (P2) or (P3), wherein the range of the ratio of solvent (or solvent mixture) to compound of formula (II) is 3:1 to 0.5 :1.

The process according to the present invention is carried out at elevated temperatures. It is usually carried out at a temperature of between 30°C and 75°C (preferably 40°C - 70°C).

Therefore the present invention relates to a process (P5), which is the process which is the process (P), (P1), (P2), (P3) or (P4), wherein the process according to the present invention is carried out at a temperature of between 30°C and 75°C (preferably 40°C - 70°C).

The c-DLL (compound of formula (I)) obtained by the process according to present invention can be purified by using commonly known processes.

As stated above the c-DLL is then used to produce 2-vinyl-2,6,6-trimethyl-3,4,5,6-tetrahydro-2H-pyran via hydrogenation.
The hydrogenation of c-DLL can be done by using commonly known hydrogenation processes. Usually it is done by catalytic hydrogenation (i.e. with Lindlar catalysts):

The following examples serve to illustrate the invention. All percentages and parts (if not otherwise stated) are related to the weight and the temperature is given in °C.

### Example 1:

50 g (0.322 mol) of dehydrolinalool (compound of formula (II)) and 50g of cyclohexane are put into a flask and stirred.
Afterwards 1.76 g of Amberlyst 15 were added under stirring and the reaction mixture was heated up by using a oil bath (oil bath temperature at 80°C). The reaction mixture was stirred for 22 hours at these reaction conditions.
The product (compound of formula (I) was obtained in a yield of 93.77 %.

## Claims

1. A process for the production of the compound of formula (I) 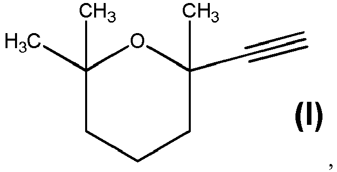 by the cyclization of the compound of formula (II) 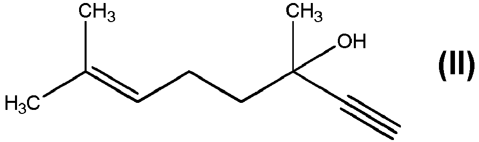 **characterized in that** the reaction is carried out in the presence of
(i) at least one acidic ion exchanger containing one or more sulfur (VI) atom as a catalyst and
(ii) at least one inert solvent,
wherein a weight ratio of 20:1 to 100:1 of compound (II) to acidic ion exchanger is used.

2. Process according to claim 1, wherein the solvent (or solvents) is chosen from the group consisting of aliphatic hydrocarbons and aromatic hydrocarbons.

3. Process according to anyone of the preceding claims 1 - 2, wherein the solvent (or more than one) is chosen from the group consisting of n-hexane, cyclohexane, n-heptane, pentane and toluene.

4. Process according to anyone of the preceding claims 1 - 3, wherein the weight ratio of solvent (or solvent mixture) to compound of formula (II) is 3:1 to 0.5 :1.

5. Process according to anyone of the preceding claims, wherein the process according to the present invention is carried out at a temperature of between 30°C and 75°C.

## Patentansprüche

1. Verfahren zur Herstellung der Verbindung der Formel (I) 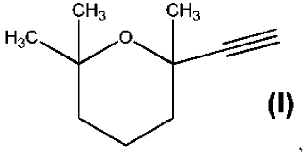 durch die Cyclisierung der Verbindung der Formel (II) 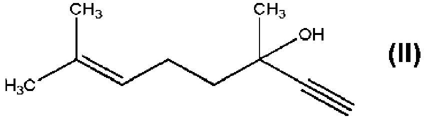 **dadurch gekennzeichnet, dass** die Umsetzung in Gegenwart von
(i) mindestens einem sauren Ionenaustauscher, der ein oder mehrere Schwefel(VI)-Atome enthält, als Katalysator und
(ii) mindestens einem inerten Lösungsmittel, durchgeführt wird,
wobei man ein Gewichtsverhältnis von Verbindung (II) zu saurem Ionenaustauscher von 20:1 bis 100:1 verwendet.

2. Verfahren nach Anspruch 1, wobei man das Lösungsmittel (oder die Lösungsmittel) aus der Gruppe bestehend aus aliphatischen Kohlenwasserstoffen und aromatischen Kohlenwasserstoffen auswählt.

3. Verfahren nach einem der vorhergehenden Ansprüche 1-2, wobei man das Lösungsmittel (oder mehr als ein Lösungsmittel) aus der Gruppe bestehend aus n-Hexan, Cyclohexan, n-Heptan, Pentan und Toluol auswählt.

4. Verfahren nach einem der vorhergehenden Ansprüche 1-3, wobei das Gewichtsverhältnis von Lösungsmittel (oder Lösungsmittelgemisch) zu Verbindung der Formel (II) 3:1 bis 0,5:1 beträgt.

5. Verfahren nach einem der vorhergehenden Ansprüche, wobei man das Verfahren gemäß der vorliegenden Erfindung bei einer Temperatur zwischen 30 °C und 75 °C durchführt.

## Revendications

1. Procédé de production du composé de formule (I) 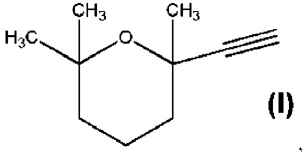 par cyclisation du composé de formule (II) 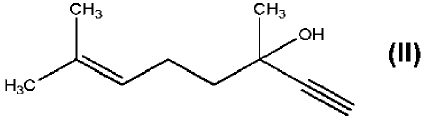 **caractérisé en ce que** la réaction est conduite en présence de
(i) au moins un échangeur d'ion acide contenant un ou plusieurs atomes de soufre (VI) en tant que catalyseur et
(ii) au moins un solvant inerte,
dans lequel un rapport en poids de 20:1 à 100:1 du composé (II) à l'échangeur d'ion acide est utilisé.

2. Procédé selon la revendication 1, dans lequel le solvant (ou les solvants) est choisi dans le groupe constitué d'hydrocarbures aliphatiques et d'hydrocarbures aromatiques.

3. Procédé selon l'une quelconque des revendications précédentes 1 et 2, dans lequel le solvant (ou plus d'un) est choisi dans le groupe constitué des n-hexane, cyclohexane, n-heptane, pentane et toluène.

4. Procédé selon l'une quelconque des revendications précédentes 1 à 3, dans lequel le rapport en poids du solvant (ou mélange de solvants) au composé de formule (II) est de 3:1 à 0,5:1.

5. Procédé selon l'une quelconque des revendications précédentes, le procédé selon la présente invention étant conduit à une température comprise entre 30 °C et 75 °C.
